# EUROPEAN PATENT SPECIFICATION

(11) **EP 1 131 457 B1**
(45) Date of publication and mention of the grant of the patent: **06.10.2010**
(21) Application number: 99959034.2
(22) Date of filing: 19.11.1999
(51) Int. Cl.: C12N 15/86, C12N 15/87, A61K 48/00, A61K 47/48, A61K 38/17

(54) **SYSTEMIC VIRAL/LIGAND GENE DELIVERY SYSTEM AND GENE THERAPY**
SYSTEMISCHES VIRUS/LIGAND GENABGABEMITTEL UND GENTHERAPIE
SYSTEME D'APPORT GENIQUE SYSTEMIQUE DE VIRUS/LIGANDS ET DE THERAPIE GENIQUE

(30) Priority: 19.11.1998 US 109236 P; 08.04.1999 US 128330 P
(43) Date of publication of application: 12.09.2001
(73) Proprietor: GEORGETOWN UNIVERSITY, Washington, D.C. 20057 (US); SynerGene Therapeutics, Inc., Potomac, MD 20854-3963 (US)
(72) Inventor: CHANG, Esther, H., Potomac, MD 20854 (US); PIROLLO, Kathleen, Rockville, MD 20850 (US); XU, Liang, Ann Arbor, MI 48103 (US); ALEXANDER, William, Rockville, MD 20853 (US)
(74) Representative: Daniels, Jeffrey Nicholas
(86) International application number: PCT/US1999/027365
(87) International publication number: WO 2000/029600

(56) References cited:
- WO-A-94/24299
- WO-A-97/38723
- WO-A-98/40508
- MILLER N ET AL: "TARGETED VECTORS FOR GENE THERAPY" FASEB JOURNAL,US,FED. OF AMERICAN SOC. FOR EXPERIMENTAL BIOLOGY, BETHESDA, MD, vol. 9, no. 2, 1 February 1995 (1995-02-01), pages 190-199, XP000616414 ISSN: 0892-6638
- DEONARAIN M P: "LIGAND-TARGETED RECEPTOR-MEDIATED VECTORS FOR GENE DELIVERY" EXPERT OPINION ON THERAPEUTIC PATENTS,GB,ASHLEY PUBLICATIONS, vol. 8, no. 1, January 1998 (1998-01), pages 53-69, XP002910260 ISSN: 1354-3776
- XU L ET AL: "Transferrin-liposome-mediated p53 sensitization of squamous cell carcinoma of the head and neck to radiation in vitro" HUMAN GENE THERAPY,XX,XX, vol. 8, no. 4, 1 March 1997 (1997-03-01), pages 467-475-475, XP002098089 ISSN: 1043-0342

## Description

### BACKGROUND OF THE INVENTION

### 1. Technical Field

The present invention relates to improvements to gene transfer and gene therapy technology. More specifically, the invention provides compositions and methods for targeted *in vitro* and *in vivo* viral delivery of nucleic acids into human and other animals to a specific organ, tissue, or tumor. The use of this invention to deliver a therapeutic gene, e.g., wtp53, can result in increased sensitivity to conventional radiation and chemotherapies.

### 2. Description of the Background Art

WO98/40508 describes adenoviral vectors with modified tropism.

Miller and Vile (1998) discloses targeted vectors for gene therapy.

Snitkovsky and Young discloses cell-specific viral targeting mediated by a soluble retroviral receptor-ligand fusion protein.

Gene delivery and gene therapy using viral vectors have been the subject of considerable research. A long-standing goal in gene therapy for cancer is a systemic delivery system that selectively targets tumor cells, including metastases. Nucleic acids can be introduced into cells via viral vectors in order to produce a desired therapeutic effect upon those cells. For example, a gene can be introduced to replace a defective gene that interferes with cell function, e.g., p53. Nucleic acids may also be introduced into cells in order to produce a desired therapeutic effect in the host animal, e.g., for vaccination, immunotherapy, or anti-sense expression.

Viral vectors have been developed to take advantage of the cell entry mechanisms used by viruses to transfer their nucleic acids into host cells. Recombinant retroviral and adenoviral vectors have been constructed using this strategy to achieve gene transfer *in vitro* and *in vivo.* Approximately 80% of the gene therapy protocols that have been approved for clinical trial utilize viral vectors (Wivel and Wilson, 1998). Adenoviral vectors offer advantages for some forms of gene therapy because they can enter non-dividing cells and carry a relatively large (>8 kb) payload of foreign DNA (Berkner, 1988). Moreover, adenoviral particles can be purified and produced in titers greater than 10¹¹ PFU/mL (Wivel and Wilson, 1998). One disadvantage of these systems, however, is the limited cell tropism of the viruses, and the significant problem of targeting viral particles has yet to be solved for any of the therapeutic viruses currently being used in clinical trials for cancer. Accordingly, methods to alter cell tropism have been developed and continue to be sought.

A system which changes the tropism of retroviruses by means of a bifunctional conjugate has been described (Roux et al., 1989). The bifunctional conjugate contains an antibody directed against the viral coat and, on the other end, an antibody directed to a specific cell membrane marker for the target cell.

Goud et al. (1988) described bifunctional conjugates which consist of two monoclonal antibodies (MAbs). The MAbs were directed against the gp70 coat protein of the Moloney retrovirus and the human transferrin receptor. These conjugates allowed the retrovirus to penetrate into the otherwise non-permissive target cells.

WO 92/06180 (Wu et al., 1992) describes a method for changing the tropism of a virus by providing the surface of a virus with a molecule that binds to a target cell surface receptor, producing a virus with a specificity for cells with the cell surface receptor. In the disclosure a retrovirus or hepatitis B virus is chemically modified with carbohydrate molecules which bind to the asialoglycoprotein receptor. Wu et al. disclose only *in vitro* methods for the introduction of foreign genes into cells.

Adenoviral vectors offer advantages for some forms of gene therapy because they can enter non-dividing cells and can carry a foreign DNA sequence of about 8 kb. Adenovirus particles can be purified and produced in titers greater than 10¹¹ PFUs/mL.

One restriction on the use of recombinant adenoviruses is their limited ability to target specific cell types. A number of studies have reported gene transfer using non-recombinant adenoviruses with DNA complexes through receptor-mediated endocytosis, with the adenovirus providing the ability to release the contents of endosomes (Cotten et al., 1992; Wagner et al., 1992). These procedures use transferrin-polylysine/DNA complexes to internalize the bound or unbound adenoviruses. Wagner et al. (1992) modified an adenoviral vector by conjugating to polylysine and complexed this with conjugates of transferrin-polylysine/DNA, producing ternary transferrin-polylysine/adenovirus-polylysine/DNA complexes. A similar approach to targeting is the linking of transferrin (Tf) to adenovirus particles to take advantage of the fact that the transferrin receptor (TfR) is elevated on many tumor types (Miyamoto et al., 1994; Baselga and Mendelsohn, 1994). Schwarzenberger et al. (1997) describe molecular conjugate vectors (MCVs). MCVs are constructed by condensing a plasmid containing the gene of interest with polylysine (PL), PL linked to a replication-incompetent adenovirus (endosomolytic agent), and PL linked to streptavidin for targeting with biotinylated ligands. However, it has been reported (Cotten et al., 1992; Wagner et al., 1992; Schwarzenberger et al., 1997) that current methods of covalent coupling of Tf to the adenovirus, or the generation of Tf-polylysine-adenovirus conjugates often results in decreased infectivity, possibly due to the harsh conditions required to produce the Tf-modified viruses.

Another approach entails crosslinking the Fab fragment of a neutralizing anti-fiber or anti-knob monoclonal antibody to a ligand, such as folate or FGF2 (Rogers et al., 1997; Douglas et al., 1996). Adenoviral vectors complexed with the chimeric Fab-ligand have shown some promise in tumor localization and exhibit reduced liver toxicity *in vivo* as compared to native adenovirus.

Curiel et al., (US Patent Nos. 5,521,291 and 5,547,932) disclose multiple adenoviral-polycation conjugates for internalizing nucleic acids into eukaryotic cells. Some of these conjugates use transferrin which is bound to a substance having an affinity for nucleic acid as an internalizing factor.

Cotten et al. (US Patent No. 5,693,509) disclose adenoviruses with the reported ability to penetrate efficiently into cells into which they cannot normally penetrate, while retaining their capacity for gene expression and/or their endosomolytic properties. Transferrin is covalently bound to adenovirus particles so that they can undergo receptor-mediated endocytosis. The method oxidizes transferrin into a form which contains aldehyde groups in the carbohydrate moiety and couples the oxidized transferrin to the adenovirus under reducing conditions. It is also disclosed that it is unpredictable whether infectivity is maintained after modification.

Low et al. (US Patent Nos. 5,108,921; 5,416,016; and 5,635,382) disclose methods for enhancing transmembrane transport of exogenous molecules using ligands such as folate, biotin, thiamine, and their analogs. The methods may also employ anti-idiotypic antibodies or other molecules capable of binding to the ligand's receptors. Other ligands disclosed include niacin, pantothenic acid, riboflavin, pyridoxal, and ascorbic acid.

Xu et al. (1997) and Xu et al. (1999) found that the addition of Tf to a cationic liposome was able to increase significantly the ability of the liposomes to deliver exogenous genes, including the normal p53 gene, both *in vitro* and *in vivo.* Most significantly, they achieved highly selective targeting to a wide variety of human tumor cells growing as xenografts in nude mice.

The publications and other materials used herein to illuminate the background of the invention or provide additional details respecting the practice, are incorporated by reference, and for convenience are respectively grouped in the appended List of References.

### SUMMARY OF THE INVENTION

The present invention provides improvements to the administration of viral vectors for, for example, viral-mediated gene delivery to target cells.

In one aspect, the invention provides a cell-targeting ligand-virus complex for delivery of a virus to a target cell within a host animal, wherein said ligand-virus complex consists of a ligand and a virus, and wherein the ligand is non-covalently bound to the virus, and is transferrin, a transferrin receptor antibody, a fragment of a transferrin receptor antibody, EGF or VEGF.

Further the present invention provides a vector for the systemic delivery of a virus to a target cell, said vector comprising a cell-targeting ligand non-covalently bound to said virus, wherein said vector is preparable by mixing a composition consisting of a cell-targeting ligand with a virus in an aqueous medium and wherein said ligand consists of transferrin, a transferrin receptor antibody, a fragment of a transferrin receptor antibody, EGF or VEGF.

Still further the present invention provides a method for preparing a vector for the systemic delivery of a virus to a target cell, said vector comprising a cell-targeting ligand non-covalently bound to said virus, comprising mixing a composition consisting of said cell-targeting ligand with said virus in an aqueous medium, whereby said ligand non-covalently binds to said virus, wherein said ligand consists of transferrin, a transferrin receptor antibody, a fragment of a transferrin receptor antibody, EGF or VEGF.

The disclosed method of producing the admixture avoids inactivation of viral particles that can otherwise be caused by harsh chemical processing. The admixture is prepared in a simple manner and is suitable for systemic (e. g., parenteral) administration to a human patient.

In another aspect, the invention provides a cell-targeting ligand-virus complex or vector as described above for use as a medicament.

Further the present invention provides a vector for use in delivery of a virus to a target cell within an animal, wherein the vector comprises a cell targeting ligand-virus complex, wherein said ligand-virus complex consists of a ligand and a virus, and wherein the ligand is non-covalently bound to the virus and is transferrin, a transferrin receptor antibody, a fragment of a transferrin receptor antibody, EGF or VEGF.

These are suitable for administering systemically in vivo to a human or other animal, so as to accomplish targeted delivery of the contents of the viral particle.

The present invention further provides the use of a virus for the manufacture of a therapeutic agent comprising a vector which comprises a cell targeting ligand-virus complex, for delivery of said virus to a target cell within an animal, wherein said ligand-virus complex consists of a ligand and a virus, and wherein the ligand is non-covalently bound to the virus and is transferrin, a transferrin receptor antibody, a fragment of a transferrin receptor antibody, EGF or VEGF.

In a preferred aspect the said vector encodes wild-type p53 and said therapeutic agent is to be administered to an animal receiving radiation treatment or chemotherapy in addition to said therapeutic agent. In this aspect, the use of the invention to deliver a therapeutic gene, wild-type p53, will lead to sensitization to radiation and chemotherapeutic agents.

### BRIEF DESCRIPTION OF THE FIGURES

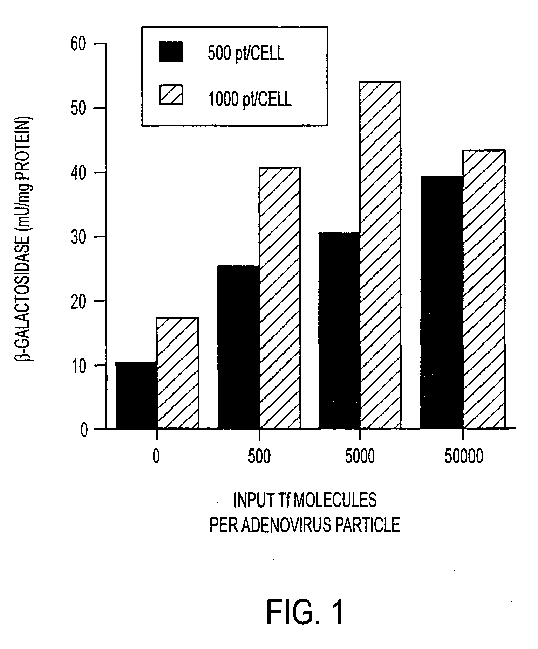

Figure 1. β-galactosidase reporter gene expression in JSQ-3 cells after infection with Tf-targeted adenoviral vector carrying the lacZ gene. 5x10⁴ JSQ-3 cells/well were plated in a 24-well plate. 24 hours later the cells were washed once with EMEM without serum and 0.3 mL EMEM without serum or antibiotics was added to each well. The Ad5LacZ or Tf-Ad5LacZ complexes at different ratios of transferrin to virus in 200 µL EMEM were added to duplicate wells. Ratios of 5x10² to 5x10⁵ Tf molecules/virion were used. The virus to cell ratios were 500 and 1000 viral particles/cell (pt/cell). After 4 hours incubation at 37°C, 5% CO₂, with mixing by rotating the test tube once every 2 minutes, 0.5 mL EMEM with 20% serum was added to the wells. After 2 days in culture, the cells were washed once with PBS, and lysed in 1 X reporter lysis buffer (Promega). The cell lysates were treated with 100 µL of 150 µM O-nitrophenyl-β-galactopyranoside in 20 mM Tris (pH 7.5) containing 1 mM MgCl₂ and 450 µM β-mercaptoethanol at 37°C for 30 minutes. The reaction was stopped by the addition of 150 µL/well of 1M Na₂CO₃ and the absorbance was measured at 405 nm. Purified β-galactosidase was used to make a standard curve. The results were expressed as milliUnit (mU) of β-galactosidase equivalent per mg of total protein.

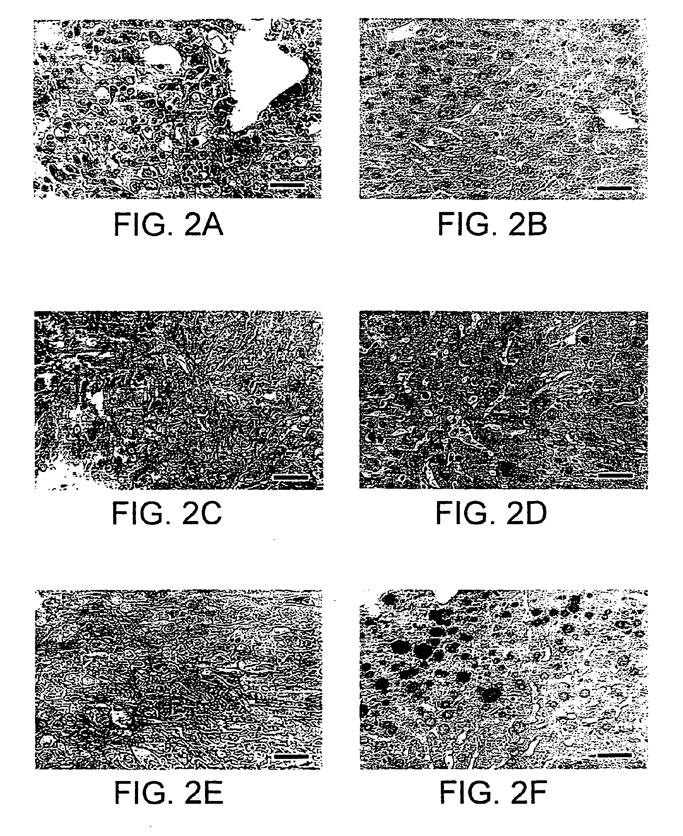

Figures 2A-F. Histochemical analysis of Tf-targeted adenoviral, systemic delivery of the β-galactosidase reporter gene in a mouse xenograft model. Athymic nude mice carrying DU 145 xenograft tumors were i.v. injected one time with Tf-AdLacZ. Three days after injection, the animals were euthanized, and the tumor and normal tissues were excised and stained with X-gal. Figure 2A shows tumor from an animal systemically treated with Tf-Adp53 at a ratio of 1.5 x 10⁵ Tf molecules/virion. Figure 2B shows liver corresponding to Figure 2A. Figure 2C shows tumor from an animal systemically treated with Tf-Adp53 at a ratio of 2.9 x 10⁵ Tf molecules/virion. Figure 2D shows liver corresponding to Figure 2C. Figure 2E shows tumor from an animal systemically treated with Tf-Adp53 at a ratio of 5.8 x 10⁵ Tf molecules/virion. Figure 2F shows liver corresponding to Figure 2E. Bar = 50 µm.

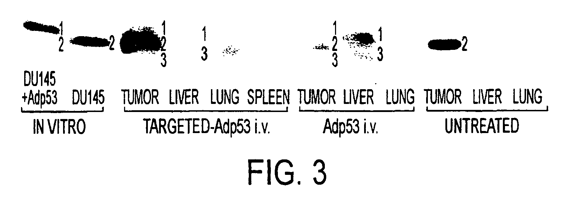

Figure 3. Exogenous wtp53 expression in DU145 xenograft tumors after i.v. injection of Tf-Adp53. Athymic nude mice carrying DU145 xenograft tumors were i.v. injected with either Tf-Adp53 or untargetted Adp53. 48 hours later the animals were euthanized, the tumor and normal tissues excised, and protein isolated for Western blot analysis. The protein isolated from the tumor and organs of an untreated mouse, as well as the parental DU145 cells, were included as controls. 100 µg of total protein of each of these samples was loaded/lane. 2.5 µg total protein of DU145 cells infected *in vitro* with Adp53 was also included. The p53 protein bands were detected using the monoclonal anti-p53 antibody Ab-2 and the ECL Western blot kit. Band 1 = Exogenous human wtp53; Band 2 = Endogenous human DU145 p53; Band 3 = Endogenous mouse p53.

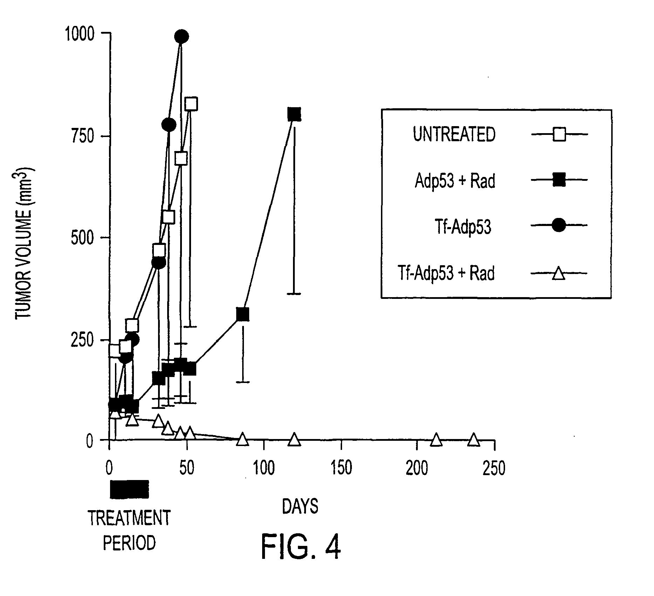

Figure 4. Effect of the combination of systemically delivered, tumor-targeted adenoviral-p53 and radiation treatment on JSQ-3 xenograft tumors *in vivo.* Tf-Adp53 was produced at a ratio of 1 x 10⁵ Tf molecules/virion. 1 x 10¹⁰ viral particles/mouse/injection (equivalent to 3 x 10⁸ pfu) of Tf-Adp53 or untargetted Adp53 were injected into the tail vein of athymic nude mice carrying JSQ-3 xenograft tumors of 100-200 mm³. Beginning the day after the first i.v. injection, 30 Gy of ionizing radiation was administered to the animals at the site of the tumor in 2 Gy daily fractionated doses. No tumor regrowth in the animals receiving the combination treatment was observed 8 months after cessation of treatment. As the error was too small to be visualized, no error bars are present in the Tf-Adp53 (+) Radiation group. The bar represents the duration of treatment (approximately 3 weeks). All animal experiments were performed in accordance with Georgetown University Institutional Guidelines for the care and use of laboratory animals.

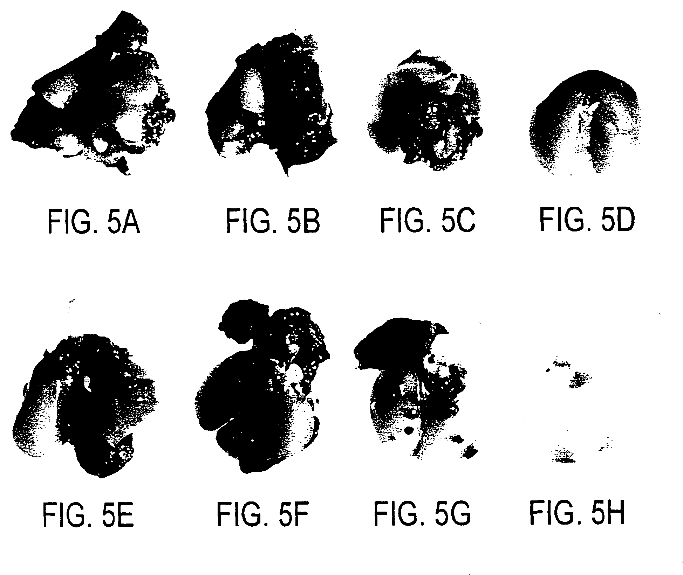

Figures 5A-H. Chemosensitization of B₁₆ mouse lung metastases to cisplatin (CDDP) by systemically delivered, tumor-targeted adenoviral-p53. The metastases were induced in normal, syngeneic C57/B1/6 mice by the intravenous injection of 1 x 10⁵ cells. Four days later treatment was begun. Tf-Adp53 and control Tf-AdLacZ were produced at the ratio of 1.5 x 10⁵ Tf molecules/virion. 1 x 10¹⁰ viral particles/mouse/injection (equivalent to 3 x 10⁸ pfu) of either Adp53, Tf-Adp53 or Tf-AdLacZ were i.v. administered 3 times/week to a total of 12-13 doses. CDDP was intraperitoneally injected at 3-5mg/kg every 2-4 days for a total of 8-13 doses. The lungs were excised from the animals after one round of treatment. Lungs from animal treated with: No treatment (Figures 5A and 5E); CDDP alone (Figure 5B); untargetted Ad-p53 plus CDDP (Figure 5C); Tf-LacZ plus CDDP (Figure 5F); Tf-Adp53 alone (Figure 5G); Tf-Adp53 plus CDDP (Figures 5D and 5H).

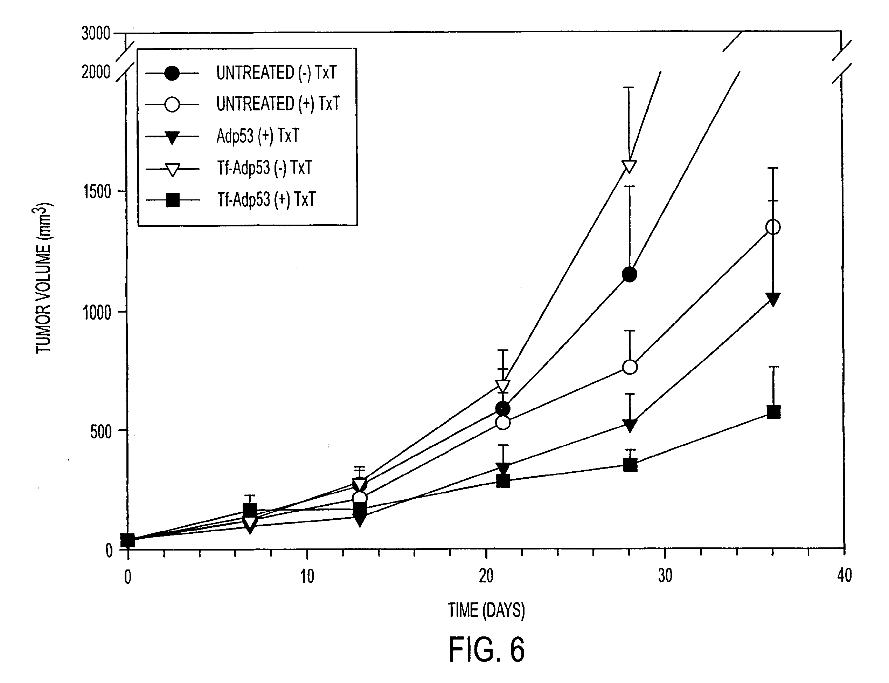

Figure 6. Effect of the combination of systemically delivered, tumor-targeted adenoviral-p53 and chemotherapy on MDA-MB-435 xenograft tumors *in vivo.*

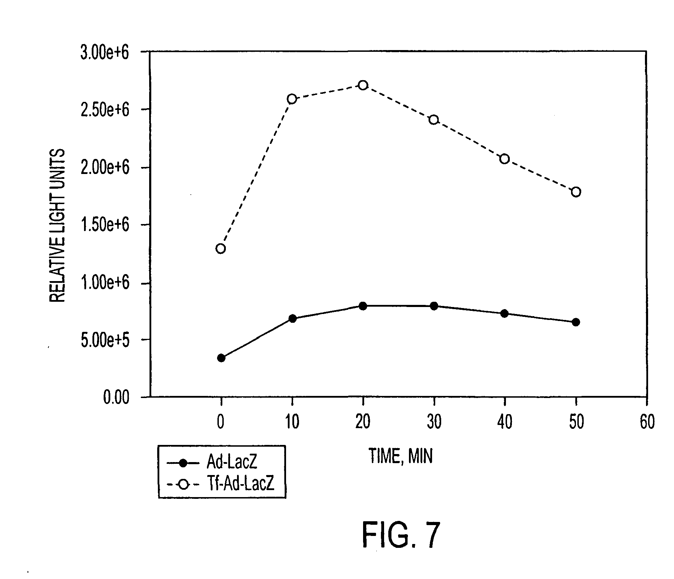

Figure 7. Expression of β-galactosidase in intratumorally injected DU145 cells which were injected with untargeted adenovirus with a LacZ or with transferrin targeted adenovirus with LacZ.

### DETAILED DESCRIPTION OF THE INVENTION

The normal development of mice lacking wtp53 and the observations of a post-irradiation G1 block in p53-expressing cells suggests that wtp53 functions in the regulation of the cell after DNA damage or stress rather than during proliferation and development. Since it appears that many conventional anti-cancer therapies (chemotherapeutics and radiation) induce DNA damage and appear to work by inducing apoptosis, alterations in the p53 pathway could conceivably lead to failure of therapeutic regimens.

Lack of wtp53 function has also been associated with an increase in radiation resistance. The presence of mtp53 and the consequent absence of a G1 block have also been found to correlate with increased radiation resistance in some human tumors and cell lines. These include human tumor cell lines representative of head and neck, lymphoma, bladder, breast, thyroid, ovary and brain cancer.

Based on these considerations, gene therapy to restore wtp53 function in tumor cells should re-establish the p53-dependent cell cycle checkpoints and the apoptotic pathway thus leading to the reversal of the chemo-/radio-resistant phenotypes. Consistent with this model, chemosensitivity, along with apoptosis, was restored by expression of wtp53 in non-small cell lung carcinoma mouse xenografts carrying mtp53. Chemosensitivity of xenografts involving the p53-null lung tumor cell line H1299 and T98G glioblastoma cells and sensitivity of WiDr colon cancer xenografts to cisplatin has been demonstrated. Increased cell killing by doxorubicin or mitomycin C was also shown in SK-Br-3 breast tumor cells by adenoviral transduction of wtp53. However, some conflicting reports indicate that the relationship between p53 expression and chemoresistance may have a tissue or cell type-specific component. The transfection of wtp53 by an adenoviral vector has also been shown to sensitize ovarian and colo-rectal tumor cells to radiation. It has also been reported that adenoviral-mediated wtp53 delivery did restore functional apoptosis in a radiation-resistant squamous cell carcinoma of the head and neck (SCCHN) tumor line resulting in radiosensitization of these cells *in vitro.* More significantly, the combination of intratumorally injected adeno-wtp53 and radiation led to complete and long-term tumor regression of established SCCHN xenograft tumors.

The current invention departs from the conventional use of intratumoral injection of untargetted viral vectors or even systemic delivery of untargetted vectors, for the delivery of therapeutic molecules for gene therapy, for example as disclosed by Roth et al. (U.S. Patent No. 5, 747,469).

The data presented herein demonstrates the superior ability of such complexes to specifically target and sensitize tumor cells (due to expression of the wtp53 gene), both primary and metastatic tumors, to radiation and/or chemotherapy both *in vitro* and *in vivo.*

The present invention addresses the need to deliver therapeutic molecules systemically with a high degree of target cell specificity and high efficiency. When systemically administered, this delivery system is capable of reaching, and specifically targeting, metastatic as well as primary disease, when the target cells are human cancer cells. As a result of delivery of the normal, wild type version of the tumor suppressor gene p53 by means of this system, the inventors demonstrated that the tumors are sensitized to radiation therapy and/or chemotherapy. The high transfection efficiency of this system results in such a high degree of sensitization that not only is there growth inhibition of the cancer but pre-existing tumors and metastases are completely eliminated for an extended period of time.

Specific embodiments of the description provide pharmaceutically acceptable compositions comprising an admixture of transferrin ligand and viral vector particles capable of delivering a nucleic acid to target host cells. Simply admixing viral vectors (e.g., retroviral or adenoviral vectors) with a cell-targeting ligand in a suitable vehicle such as sterile-water-for-injection increases transfection efficiency over that obtained with the viral vector alone. A simple admixture of viral vector and transferrin, for example, increases the transfection of cells, e.g., human cancer cells, expressing the transferrin receptor.

The use of transferrin is especially advantageous in connection with gene transfer into, or gene therapy for, a wide variety of human cancers. A wide variety of human cancer cells contain transferrin receptors. The presence of transferrin in the complexes and vectors of the present invention permits the viral vectors to efficiently and specifically target those cancer cells.

Other ligands, such as proteins, peptides, hormones, antibodies and antibody fragments will be useful for specifically targeting the viral vectors to cells containing receptors for such ligands or which can internalize the ligand by receptor-mediated endocytosis. The ligand, for example, can be a native or recombinant protein that functions to enhance the binding of the viral vector to a target cell. Examples of ligands include insulin, toxins, EGF, VEGF, FGF, IGF, heregulin, other viral or bacterial proteins, estrogen and progesterone.

Cell targeting ligands according to the present invention are transferrin, a transferrin receptor antibody, a fragment of a transferrin receptor antibody, EGF or VEGF.

While the description envisages the use of a cell-targeting ligand which occurs naturally on one type of virus when this ligand is mixed with a second type of virus, the description does not envisage the use of a cell-targeting ligand in its naturally occurring association with the virus which encoded it. The description does envisage the mixture of a cell-targeting ligand encoded by a virus in association with the virus type which encodes said ligand when the ligand is present in an amount higher than normally found in association with the naturally occurring virus.

The method by which a complex is formed between the ligand and the viral particle is such that a large number of ligand molecules coat the surface of the viral particle and increase the stability thereof as it travels through the blood stream. Moreover, the high number of ligand molecules on the surface may also serve to decrease the immunogenicity of the virus by blocking viral antigens.

The invention includes the use of recombinant expression viruses. Viral vectors, such as recombinant adenovirus, AAV vectors (U.S. Pat. No. 5,139.941), retroviral vectors, herpes simplex virus (U.S. Pat. No. 5,288,641), cytomegalovirus (CMV), vaccinia virus, fowlpoxvirus (FPV), canarypoxvirus (CPV)(U.S. Pat. Nos. 5,833,975; 5,762,938; and 5,378,457), Sindbis virus, chimeric or hybrid viruses and the like may be used in accordance with the invention. Replication-competent or oncolytic viruses also can be used in accordance with the invention.

The invention also provides methods for preparing a viral vector-transferrin admixture which advantageously avoids the harsh chemicals and complicated processing steps that have been described in connection with previously used methods using MAbs, linkers, polylysine, etc., to link transferrin to viral vectors.

In accordance with the invention, ligand-viral admixtures can be prepared in any carrier or vehicle (typically an aqueous carrier) so as to provide a composition that is pharmaceutically suitable for *in vitro* or *in vivo* administration. The composition typically will be buffered to a suitable pH and can contain suitable auxiliary components such as osmolarity adjusting agents, antibiotics, etc.

The amounts of viral particles used in the admixtures and ultimately administered to the host animal (or administered to cells *in vitro*) will be determined by those skilled in this field based upon well-known principles of gene transfer and gene therapy described in the scientific literature. The admixtures of the invention are administered via methods analogous to those previously described for the administration of viral vectors so as to carry out *in vitro* or *in vivo* gene transfer or gene therapy. The invention improves upon existing technology by providing compositions and methods for the systemic administration of viral vectors. Parenteral administration (especially intravenous or intra-arterial administration) of the admixtures is preferred. It is anticipated that, in some cases, substantially (for example 30-fold) lower doses of viral particles can be administered due to the improved efficiency brought about by the invention. Alternatively, in other cases, known doses can be used, resulting in increased genetic transfer. Concentrations of viral particles, ligand and auxiliary agents within the compositions of the invention also will be suitably selected.

Specific embodiments of the invention provide for using the compositions of the invention in conjunction with radiation treatment and/or chemotherapy.

The invention is not limited to any particular viral vector, or to any particular route or mode of administration of the ligand-viral vector admixture compositions. The desired total dose can be determined experimentally, and can be provided to a patient in need of gene therapy in a single or in multiple administration(s).

The data presented in the Examples indicate that Tf-adenovirus complexes are capable of producing markedly higher levels of gene expression in tumors than that seen with untargetted adenoviral vectors. The gene delivery method described here is based upon the relatively simple method of producing the Tf-targeted viruses. The coupling is non-covalent and does not involve chemical reactions capable of producing unwanted and perhaps toxic side products and avoids the harsh chemical conjugation and complicated processing steps that have been described in connection with previously used methods using MAbs, linkers, polylysine, etc., to link Tf to viral vectors. These chemical modifications of viruses inevitably lower the infectivity of the virus and can produce aggregates possibly too large to penetrate the tumor capillaries. While intratumorally injected viral gene therapy vectors including oncolytic viruses are in clinical trials, no targeted viruses without covalent modifications are currently under clinical study.

While it is becoming evident that single agent p53 gene therapy is not sufficient to completely eliminate tumors long term, the presently-described combination of Tf-targeted adenovirus and conventional radiation/chemotherapy was able to achieve not only growth inhibition, but tumor regression, demonstrating a synergistic effect.

The *in vivo* studies described herein demonstrate that the combination of systemic Tf-Adp53 gene therapy and conventional radiotherapy and/or chemotherapy is markedly more effective than either treatment alone. In the clinical setting, radiation doses of 65 to 75 Gy for gross tumor and 45 to 50 Gy for microscopic disease are commonly employed in the treatment of head and neck cancer. Given the known, adverse side effects associated with high doses of radiation or chemotherapy, sensitization of tumors so as to permit a lowered effective dose of the conventional treatment would be of immense clinical benefit. Furthermore, in the case of radiation, systemic restoration of wtp53 function, resulting in a decrease in the radiation treatment dose found to be effective, would permit further therapeutic intervention for tumors which did reoccur.

The sensitization of tumors to chemotherapy and radiation will result in increased efficacy of current treatment modalities. Moreover, the potential also exists for this tumor specific combination treatment to lower the necessary dose of both types of conventional anticancer modalities thereby lessening the severe side effects often associated with these treatments. In the *in vivo* studies described in the Examples, systemic administration of Tf-Adp53 in combination with radiation resulted in total and long-term tumor regression using as little as 3 x 10⁸ pfu of the tumor-targeting virus. This dose is approximately equivalent to that employed by Kataoka et al. (1998) using untargetted Adp53 and 2-Me. In that study, partial tumor growth inhibition (two thirds reduction in lung colony count) was observed.

Although this system may well eliminate the need for intratumoral injection of gene therapy vectors, in the case of very aggressive cancers it may also be beneficial to use both systemic and intratumoral treatments. This system may also be adapted to assist in the delivery of other viral cancer treatments. For example, current trials of Onyx's oncolytic viruses do not support systemic delivery. ONYX-015 is a genetically modified adenovirus that efficiently replicates in and kills tumor cells deficient in wtp53 tumor suppressor activity ("p53-deficient" cells) and not in normal cells. The specific modification of the virus prevents it from replicating efficiently in normal cells. Clinical studies with ONYX-015 are currently underway for head and neck cancer, pancreatic cancer and ovarian carcinoma (Heise et al., 1997; Hall et al., 1998; Linke, 1998; Kirn et al., 1998). Our ability to target viruses to tumors may significantly improve the efficacy of other virally based cancer treatments such as ONYX-015.

Most significantly, systemic administration means that both the primary tumor and distant metastases can be reached with therapeutic genes. This is in stark contrast to that which can be achieved with intratumoral injection. The method described here is adaptable to targeting any existing recombinant viruses. It is also independent of the gene to be delivered. Additionally, this system could, as mentioned above, also be used with oncolytic viruses. The use of Tf for targeting is especially attractive in connection with p53 gene therapy for human cancers in that a broad spectrum of cancers express elevated levels of the TfR, and in over 50% of cancers, the p53 gene has been implicated. Therefore, these findings demonstrate the clinical potential of this Tf-targeted adenoviral delivery system as a new, more efficient and effective form of gene therapy for cancer, one which will help to fulfill the initial promise of gene therapy in the war against this disease.

Specific, illustrative embodiments of the present invention are provided in the following Examples.

### Example 1

### Transferrin enhances adenoviral transduction efficiency

### A. Preparation of Transferrin-Adenovirus Admixture

Holo-transferrin (Tf, iron-saturated, Sigma) was dissolved in sterile water at 5 mg/mL. Replication deficient adenovirus serotype 5, designated Ad5LacZ (Ad5CMVntbeta-gal, Gene Transfer Vector Core, University of Iowa), containing the *E. coli* LacZ gene under control of the CMV promoter, at a concentration of 1.1x10¹² particles(pt)/mL (which contained 5.5x 10⁹ plaque forming units, pfu/mL) in PBS plus 3% sucrose, was used in the study. Tf was first diluted to 0.5 mg/mL in 10 mM HEPES buffer, pH 7.4, then the Tf was added to 50 µL HEPES buffer in a 10-fold serial dilution. Ad5LacZ was then added to the tubes so that the Tf to virus ratios ranged from 1 x 10² up to 1 x 10⁶ Tf molecules/virion. The tubes were incubated at room temperature for 10-15 minutes, with rocking (rotating the tubes once every two minutes), and then 150 µL EMEM without serum was added to each tube.

### B. In vitro transduction using adenovirus/Tf admixture

We have employed a replication deficient adenovirus of serotype 5 termed AdLacZ (containing the *E*. *coli* LacZ gene under control of the CMV promoter) and the Tf-modified form of this virus (Tf-AdLacZ). To optimize the ability of Tf-AdLacZ to deliver the reporter gene, cultures of the cell line JSQ-3 (Weichselbaum et al., 1988), derived from a human squamous cell carcinoma of the head and neck (SCCHN), were infected (Bischoff et al., 1996) with AdLacZ or with Tf-AdLacZ produced using different ratios of Tf to virus and virus particles to cell.

5 x 10⁴ JSQ-3 cells/well were plated in a 24-well plate. 24 hours later the cells were washed once with EMEM without serum, 0.3 mL EMEM without serum or antibiotics was added to each well. The Ad5LacZ or Tf-Ad5LacZ complexes at different ratios of transferrin to virus in 200 µL EMEM were added to duplicate wells. The virus to cell ratio ranged from 20 up to 2000 viral particles/cell (pt/cell). After 4 hours incubation at 37°C. 5% CO₂, with occasional rocking, 0.5 mL EMEM with 20% serum was added to the wells. After 2 days in culture, the cells were washed once with PBS, and lysed in 1X reporter lysis buffer (Promega). The cell lysates were treated with 100 µL of 150 µM O-nitrophenyl-β-galactopyranoside in 20 mM Tris (pH 7.5) containing 1 mM MgCl₂ and 450 µM β-mercaptoethanol at 37°C for 30 minutes. The reaction was stopped by the addition of 150 µL/well of 1 M Na₂CO₃. The absorbance was measured at 405 nm. Purified β-galactosidase (Boehringer) was used to make a standard curve. The results were expressed as milliUnits (mU) of β-galactosidase equivalent per mg of total protein.

### C. Histochemical staining

For histochemical studies of Tf-Ad5LacZ transduction, 60% confluent cells in 24-well plates were transfected for 5 hours with transfection solutions as described above. After an additional 2 days in culture, the cells were fixed and stained with X-gal (Xu et al., 1997). Transfection efficiency was calculated as the percentage of blue-stained cells.

### D. Results and Discussion

At certain ratios of Tf to virus or of virus to cell, expression with the Tf-AdLacZ was between 3- to 4-fold higher than that seen with the untargetted AdLacZ (see Figure 1). At a viral dose of 500 pt/cell or 2.5 MOl (multiplicity of infection, or pfu/cell), 10 mU/mg protein of reporter gene product β-galactosidase was expressed by Ad5LacZ alone. Transduction with the transferrin-virus admixture Tf-Ad5LacZ (500 Tf molecules/pt) produced 25 mU/mg protein of reporter gene expression, Tf-Ad5LacZ (5,000 Tf molecules/pt) produced 30 mU/mg expression, and Tf-Ad5LacZ (50,000 Tf molecules/pt) produced 38.8 mU/mg expression, which represents 2.5, 3, and 3.8-fold, respectively, more gene transduction than attained with Ad5LacZ alone. At a dose of 1,000 pt/cell or 5 MOI, Tf-Ad5LacZ (500 Tf molecules/pt) gave 2.4-fold more reporter gene expression and Tf-Ad5LacZ (5000 Tf molecules/pt) gave 3.3-fold more gene expression than Ad5LacZ only. Tf-Ad5LacZ (50,000 Tf molecules/pt) gave 2.6-fold more expression, seeming to reach saturation. Therefore, the optimal ratio of Tf-Ad5LacZ complex appeared to be about 500-50,000 Tf molecules/pt, preferably about 5000 Tf molecules/pt. It is thought that the large number of transferrin molecules used to coat the surface of the viral particles increased its stability as it traveled through the bloodstream. The large number of transferrin molecules on the virion surface can also serve to decrease the immunogenicity of the virus by blocking viral antigen exposure.

Histochemical staining showed that Ad5LacZ alone gave 20-30% transduction efficiency while transferrin complexed adenovirus Tf-Ad5LacZ (5000 Tf molecules/pt) gave 70-90% efficiency.

The above results demonstrated that adenoviral-transferrin admixtures can substantially enhance adenoviral gene transduction.

### Example 2

### Transferrin-targeted Systemic Adenoviral

### Gene Delivery in Nude Mouse JSQ-3 Xenograft Model

### A. Preparation of transferrin-adenovirus complex

The transferrin-Ad5LacZ complex was prepared by means similar to those used for the preparation described in Example 1. 1 x 10⁹- 1 x 10¹⁰ pt Ad5LacZ (1x10¹²pt/mL in PBS plus 3% sucrose) was mixed with different amounts of Tf (4 to 5 mg/mL in water) at ratios ranging from 1 µg to 1 mg Tf/1x10¹⁰ pt, or 7.5x10²-7.5x10⁵ Tf molecules/virion. The mixtures were incubated at room temperature for 5-10 minutes with rocking (rotation of the tubes once every two minutes) to permit the Tf-Ad5LacZ complex to form. PBS (pH 7.4) was added to each tube to dilute to 1x10⁹-1x10¹⁰pt/0.2-0.3 mL/mouse injection.

Two types of human tumors were established as xenografts in nude mice by subcutaneous injection of either the SCCHN cell line used in the culture experiments above (JSQ-3) or the human prostate cancer cell line DU 145 (Isaacs et al., 1991; Asgari et al., 1997). The nude mouse tumor model was established by subcutaneous injection of JSQ-3 cells or DU 145 cells into the flank of 4-6 week old female nude mice (Xu et al., 1997). The tumors were allowed to grow to a size of 1-2 cm³. 1 x 10⁹- 1 x 10¹⁰ pt Ad5LacZ complexed with different amounts of Tf in 200-300 µL were injected into each mouse via the tail vein with a 1 cc syringe and a 30 G needle. In the control group, Ad5LacZ was injected. Three days after injection, the tumors as well as mouse organs were excised, cut into 1 mm sections, washed once with PBS, and fixed with 2% formaldehyde/0.2% glutaraldehyde for 4 hours at room temperature. The fixed tumor sections were washed 4 times, each for 1 hour, and stained with X-Gal solution plus 0.1% NP-40 (pH 8.5) at 37°C overnight. The stained tumor sections were embedded and sectioned using normal histological procedures and counter-stained with nuclear fast red. Four sections per tumor were examined to evaluate the β-galactosidase gene expression, as indicated by the blue stained cells.

Another tumor section was used for quantitative β-galactosidase assay. The tissues were homogenized and lysed in 1 X reporter lysis buffer (Promega). The lysates were added to a 96-well plate and a quantitative β-galactosidase assay was carried out as described in Example 1. In some experiments, the quantitative β-galactosidase assay was also performed using a Luminescent β-galactosidase Detection Kit II (Clontech).

### B. Results and Discussion

To test systemic, targeted viral gene delivery, viral vectors were injected i.v. in well-established solid tumor models. Two solid tumor xenograft models were used to test the targeted viral gene delivery system. 1 x 10¹⁰ pt Ad5LacZ alone or complexed with different amounts of Tf were i.v. injected into nude mice bearing 1-2 cm³ size human tumor xenografts of JSQ-3 and DU145 cells. Three days later, the tumors injected with Tf-Ad5LacZ showed increased X-Gal-stained blue cells, as compared with Ad5LacZ alone (<1%). The efficiency increased as a result of increased Tf/pt ratios, from 5% up to >35% (0.01 mg-0.6 mg/10¹⁰pt). The efficiency started to decrease with Tf/pt ratios >0.6-1 mg/10¹⁰pt (about 4.5 to 6 x 10⁵ Tf molecules per virion). This is shown in Figures 2A, 2C, and 2E where the percent of β-galactosidase expressing cells (as indicated by X-gal staining) actually decreases as the ratio of Tf-molecules/virion increases from 2.9 x 10⁵ Tf/virion to 5.8 x 10⁵ Tf/virion (Figures 2C and 2E). Moreover, at the ratio of 1.5 x 10⁵ Tf/virion no β-galactosidase expression was evident in the liver (Figure 2B) or other organs including spleen and lung, while there was minimal, but clearly detectable, β-galactosidase expression in the liver at the ratio of 2.9 x 10⁵ Tf molecules/virion (Figure 2D). In contrast, injection of Tf-AdLacZ produced with higher ratios of Tf/virion - resulted in notable liver staining (Figure 2F). Therefore, based upon these findings, the ratio of 1.5 x 10⁵ Tf molecules/virion, which demonstrated significant tumor transfection efficiency, while maintaining the highest degree of tumor specificity was determined to be optimal for the studies. The optimal conditions appeared to be about 0.01-0.2 mg/10¹⁰ pt (7.5x10³-1.5x10⁵ Tf molecules per virion) for JSQ-3 and about 0.03-0.5 mg/10¹⁰ pt for DU 145. Therefore, Tf/pt ratios can be optimized *in vivo* in different tumor models. It should be noted that *in vitro* optimal Tf/pt ratios are much smaller than that of *in vivo,* such that more transferrin may be needed *in vivo* to stabilize the virus for improved targeting. The 0.2 mg Tf/10¹⁰ pt (1.5x10⁵ Tf molecules per virion) ratio was used in subsequent *in vivo* gene therapy experiments for JSQ-3 tumors.

Quantitative β-galactosidase assays also confirmed the substantial increase of gene expression in tumors of mice i.v. injected with transferrin-targeted adenovirus, compared with that of adenovirus alone.

Targeted organ delivery of virus was also observed in livers of mice with i.v. injected Tf-Ad5LacZ complex, but liver delivery has a different preferred Tf/pt ratio, e.g. about 0.5 mg-1.3 mg Tf/10¹⁰ pt (3.75x10⁵-9.75x10⁵ Tf molecules per virion). In Ad5LacZ alone i.v.-injected mice, only a limited number of hepatocytes stained blue (<1-5%), while mice i.v.-injected with Tf-Ad5LacZ showed increased blue hepatocytes (10%-40%). The difference of preferred Tf/pt ratios between tumor-targeting and liver-targeting illustrates that systemic viral delivery systems according to the present invention can be optimized so as to be selective for different targets.

### Example 3

### Transferrin-targeted Adenoviral-Mediated Gene Delivery and Protein Expression of p53 In Vivo in a DU 145 Xenograft Nude Mouse Model

The ability of the transferrin-targeted adenoviral vector to deliver the p53 gene selectively to tumors was examined. The replication deficient adenovirus serotype 5, carrying the normal human p53 gene was used in these studies. This virus, termed Adp53, was used to produce Tf-Adp53. Tf-Adp53 was produced by mixing Holo-Transferrin with Adp53 in 10 mM HEPES, pH 7.4, at a ratio of 1.5 x 10⁵ Tf molecules/virion. After incubation for 10 minutes at 4°C, phosphate buffered saline (PBS), pH 7.4, was added to bring the final volume to 300 µL/mouse and made to a final concentration of 5% dextrose. Three days after i.v. injection of these viruses into nude mice bearing subcutaneous DU 145 tumors, the mice were euthanized, the tumors and organs excised, and Western blot analysis for p53 protein expression performed (see Figure 3). The antibody used in these studies reacts with both normal and mutated forms of human p53 and cross-reacts with mouse p53. The p53 band representing the virally transduced wild-type p53 migrates in the gel above the mutated form of p53 found in the DU145 cells. This can be seen in the left two lanes of Figure 3 where DU145 cells are compared with DU145 cells infected in culture with Adp53. An upper band, representing the virally encoded wtp53, was evident in DU 145 cells infected *in vitro* with Adp53. It should be noted that the first lane (DU145 + Adp53) contains only 2.5 µg of protein whereas all other lanes of Figure 3 contain 100 µg of total protein.

Western blot analysis of tumors from mice receiving the targeted Adp53 (i.e., Tf-Adp53) revealed an upper band (exogenous p53) and a lower band (endogenous DU 145 p53) that merged into what appears as a single large band. It is clear that there is significantly more exogenous p53 in tumors from mice receiving Tf Adp53 than the tumors from the mice treated with the untargetted Adp53. Liver and other vital organs from the mouse treated with the targeted Tf-Adp53 displayed little or no exogenous wtp53. In contrast, treatment with untargetted Adp53, resulted in a higher level of exogenous p53 in the liver. As would be expected, tumors of untreated mice contained only the endogenous DU 145 p53 and organs from these animals contained only endogenous mouse p53. These results further confirm that the Tf-Adp53, and not the untargetted Adp53, can selectively target tumors *in vivo,* and that p53 is efficiently expressed in the tumor tissue following i.v. administration.

### Example 4

### Transferrin-Targeted Systemic Adenoviral-Mediated Gene Delivery In Vivo in an SCCHN Xenograft Nude Mouse Model in Conjunction with Radiation Treatment

The ultimate test of the usefulness of a targeted adenoviral delivery system in gene therapy is its effectiveness in treating tumors when systemically administered. It has been established that loss of functional p53 can contribute to the radiation-resistant phenotype (Bristow et al., 1996). We previously demonstrated that the combination of wtp53 and conventional radiation treatment was able to eliminate established xenograft tumors long term (Xu et al., 1999; Pirollo et al., 1997). The results in this example show the ability of Tf-Adp53 to sensitize xenografts of human tumor cells to radiation therapy.

Xenografts were induced in 4-6 week old female athymic nude (NCr nu-nu) mice by the subcutaneous injection of 4 x 10⁶ JSQ-3 cells (in Matrigel^{™}, a collagen matrix) on the lower back above the tail of each animal. The JSQ-3 cell line was derived from a recurrent SCCHN and is known to be highly radioresistant. Tumors were allowed to develop to a size of 100-200 mm³. The targeted adenovirus, designated Targeted Ad-p53, was prepared by mixing transferrin with adenovirus carrying DNA encoding wt p53 as described in Example 1. Adenovirus particles (pt) per plaque forming unit (pfu) was calculated for this experiment. The animals were divided into four groups: (i) Untreated (-) Radiation; (ii) Untargetted Ad-p53 (+) Radiation; (iii) Targeted Ad-p53 (-) Radiation; (iv) Targeted Ad-p53 (+) Radiation. The mice (in groups ii, iii and iv) were i.v. injected, via the tail vein, every three to four days with 1 x 10¹⁰ pt/mouse/injection (equivalent to approximately 3 x 10⁸ pfu) of either targeted (an admixture of Tf ligand and virus was injected) or untargetted (no ligand) Ad-p53. A total of 6 injections were administered. The day after the initial i.v. injection, the animals (in groups ii and iv) were secured in a lead holder, which permitted only the tumor area to be irradiated, and the first fractionated dose of 2.0 Gy of ¹³⁷Cs ionizing radiation administered using a J.L. Shepard and Associates Mark I irradiator. Thereafter, the animals were given 2.0 Gy/day for 5 consecutive days, followed by 2 days without radiation treatment. The cycle was repeated until a total of 30 Gy had been administered. The tumor sizes were measured weekly in a blinded manner.

The untreated animals and those receiving Targeted Ad-p53 without radiation were euthanized due to tumor burden by day 52 (see Figure 4). Treatment with Untargetted Ad-p53 plus radiation delayed tumor growth during the course of treatment. However, once treatment ceased, the tumors in these animals began to increase in size such that by day 121 they also had to be euthanized due to tumor burden. In contrast, the tumors in the animals receiving Tf-Targeted Ad-p53 in combination with radiation regressed completely, during and even after cessation of treatment, such that more than 8 months post treatment there was no recurrence of the tumors in these animals.

These results together with those in Figure 3 demonstrate that systemically administered Tf-targeted adenovirus can deliver wt-p53 selectively to tumors resulting in their sensitization to conventional radiotherapy. Most importantly, the combinatorial treatment of Tf-Adp53 plus radiation resulted in eradication of tumors long-term. Recently, Kataoka et al. (1998) reported that the combination of 2-methoxyestradiol (2-Me) and systemic delivery of untargetted Adp53 in a mouse model using A549 cells partially inhibits metastatic lung tumor growth. This combination treatment resulted in a two-thirds reduction in lung colony count. While certainly promising, the tumor cells remaining after this treatment would most certainly grow and eventually kill the animal. In contrast, our results with Tf-targeted Adp53 produced apparently total, long-term (currently 8 months out) regression of subcutaneous SCCHN tumors.

### Example 5

### Transferrin-Targeted Systemic Adenoviral-Mediated Gene Delivery In Vivo in an SCCHN Xenograft Nude Mouse Model in Conjunction with Radiation Treatment

JSQ-3 xenografts were induced in NCr nu-nu mice as in Example 4. Tumors were allowed to develop to a size of 50-60 mm³. The targeted adenovirus, with (Targeted Ad-p53) or without (Targeted Ad) DNA encoding human wt p53, was prepared by mixing transferrin with adenovirus as described in Example 1. Adenovirus particles (pt) per plaque forming unit (pfu) was calculated. The animals were divided into five groups: (i) Untreated (-) Radiation; (ii) Untargetted Ad-p53 (+) Radiation; (iii) Targeted Ad (+) Radiation; (iv) Targeted Ad-p53 (-) Radiation; (v) Targeted Ad-p53 (+) Radiation. Mice were i.v. injected via the tail vein every three to four days with 3 x 10⁹ pt/mouse/injection. A total of 5 injections were administered. Three days after the initial i.v. injection, the animals were secured in a lead holder, which permitted only the tumor area to be irradiated, and the first fractionated dose of 2.0 Gy of ¹³⁷Cs ionizing radiation was administered using a J.L. Shepard and Associates Mark I irradiator. Thereafter, the animals were given 2.0 Gy/day for 5 consecutive days, followed by 2 days without radiation treatment. The cycle was repeated until a total of 26 Gy had been administered. The tumor sizes were measured weekly in a blinded manner. As in Example 4, the tumors in the untreated animals and those receiving Targeted Ad-p53 without radiation demonstrated continuous growth such that by day 50 the animals were euthanized due to tumor burden. Tumors in the group treated with radiation and Targeted-Ad without wt p53 demonstrated some minimal radiation inhibition of growth during treatment, but tumors increased in volume once the treatment was ended. A similar but even more dramatic regrowth occurred post-treatment in the group of animals that received the Targeted-Ad-p53 but no radiation. This is in sharp contrast to those mice receiving the Targeted Ad-p53 in combination with radiation. As observed in Example 4, tumor regression continued in these animals more than eight months after the end of all treatment. Eight months in the lifespan of a mouse is equivalent to 30 years in a human life span.

### Example 6

### Transferrin-targeted Retroviral Gene Transduction -

Retroviral vectors are one of the most widely used gene therapy vectors in clinical trials. As with adenoviral vectors, retroviral vectors exhibit poor specificity and significant immunogenicity.

Replication-deficient retrovirus containing the *E*. *coli* LacZ gene, RvLacZ (A Lac Z, Gene Transfer Vector Core, University of Iowa), at 1x10¹⁰ particles (pt)/mL containing 3x10⁷ transforming unit (TU)/mL, was employed in this study. Transferrin and Tf-RvLacZ complex was prepared similarly to that described in Example 1. Briefly, Tf was first diluted to 0.5 mg/mL in 10 mM HEPES buffer, pH 7.4, then different amounts of Tf were added to 50 µL HEPES buffer in a serial dilution. RvLacZ was then added to the tubes so that the Tf to virus ratios ranged from 1x10² up to 1x10⁶ Tf molecules/virion. The tubes were incubated at room temperature for 10-15 minutes with rocking every two minutes then 150 µL of EMEM without serum was added to each tube. *In vitro* retroviral transduction was performed as described in Example 1. The virus to cell ratio ranged from 100 to 2000 viral pt/cell.

At a viral dose of 500 pt/cell or 1.5 MOI (or TU/cell), 3.4 mU/mg protein of β-galactosidase was expressed by the retrovirus RvLacZ alone. With transferrin-complexed virus, administration of Tf-RvLacZ (500 Tf molecules/pt) gave 6.8 mU/mg protein of β-galactosidase expression, while administration of Tf-RvLacZ (5000 Tf molecules/pt) gave 9 mU/mg expression, which represents 2- and 3-fold higher gene transduction than produced via the administration of RvLacZ alone. The increase of gene transduction plateaued at a ratio of 50000 Tf molecules/pt. At a dose of 1000 pt/cell or 3 MOI, Tf-RvLacZ (5000 Tf molecules/pt) produced 2.1-fold more reporter gene expression and Tf-RvLacZ (50000 Tf molecules/pt) produced 3-fold more expression than RvLacZ alone. Histochemical staining showed that RvLacZ alone had a 20-30% transduction efficiency while transferrin-complexed retrovirus Tf-RvLacZ (5000 Tf molecules/pt) had a 60-80% transduction efficiency. The results demonstrated that the administration of an admixture of transferrin and retrovirus can substantially enhance retroviral gene transduction.

### Example 7

### Transferrin- Targeted Systemic Adenoviral-Mediated Gene Delivery In Vivo in a Syngeneic Mouse Model in Conjunction with Chemotherapy

The ability of the ligand-targeted, viral p53 delivery system to sensitize tumor cells to chemotherapy in an immune competent animal model was examined. The model chosen for these studies was the B₁₆ mouse melanoma lung metastases model. In multiple experiments, B₁₆ cells were injected, via the tail vein into immune-competent C57/BL/6 mice. In this model, tumor colonies in the lung are easily visible within two-three weeks, due to the expression of melanin by the tumor cells. Four days after injection of the B₁₆ cells, treatment with the combination of Adp53, Tf-Adp53 and/or cisplatin (CDDP) was begun. I x 10¹⁰ viral pt/mouse/injection (equivalent to 3 x 10⁸ pfu), at a ratio of 1.5 x 10⁵ Tf molecules/virion, was administered systemically via an i.v. tail vein injection three times per week for a total of 12-13 viral treatments. Intraperitoneal CDDP (3-5mg/kg) was administered every 2-4 days with a total of 8-13 doses of CDDP administered. One day after all treatments (five weeks after the initial injection of B₁₆ cells), the lungs were excised from the animals and perfused with 10% formaldehyde.

As shown in Figures 5A-H, there is a dramatic difference in the lungs obtained from the animals receiving the combination treatment and those from the other groups in two separate experiments. While CDDP alone and the Tf-Adp53 alone demonstrated some effect when compared to the lungs from the untreated animals, a significant number of tumor colonies are still evident. Similarly, the lungs of animals treated with the control virus Tf-AdLacZ plus CDDP evidences what is only a drug effect. More significantly, the animals that received the untargetted Adp53 along with CDDP also present with multiple large tumor colonies indicating minimal effect of untargetted Adp53 when systemically delivered. In contrast however, the lungs from the animals that received transferrin-targeted adenoviral p53 (Tf-Adp53) in combination with CDDP are virtually free of obvious tumor metastases. These findings demonstrate that the systemically delivered, Tf-targeted, Adp53 can sensitize tumor cells to conventional chemotherapy in addition to conventional radiotherapy. Moreover, the Tf-Adp53 can also - function effectively in a syngeneic mouse model in addition to the nude mouse models used previously.

The Tf molecule employed in all of our experiments is human Tf. It is known that the TfR from a given species can bind the Tf from a range of other species (Aisen, 1998). Nonetheless, we were initially concerned that the selectivity seen with human tumors in nude mice might be attributable to the human Tf we were using being recognized by the tumor TfR in preference to the mouse TfR in the host's normal tissues. The syngeneic model system involving B₁₆ mouse melanoma cells growing in C57/BL/6 mice is reassuring in this regard. In this model, the TfR on the tumor and the TfR on the normal tissues are both mouse TfR. Nonetheless, we were able to demonstrate that adenoviruses complexed with human Tf home to the tumors having elevated levels of mouse TfR. This finding suggests that it is the level of TfR expression rather than a species-related phenomenon that accounts for targeting to the human xenograft tumors in the nude mouse models described above and boosts the likelihood that we can achieve our ultimate goal of treating human tumors growing in humans.

### Example 8

### Transferrin Targeted Herpes Simplex Virus Transduction

Herpes simplex virus (HSV) is a replication competent viral vector, widely used in gene therapy, especially in the central nervous system (Walker, 1999). As with adenoviral or retroviral vectors, HSV exhibits poor specificity and significant immunogenicity.

To explore the feasibility of using the transferrin-targeting strategy to target the replication competent viral vectors, G207, the HSV with a reporter gene LacZ (Walker, 1999), was complexed with human transferrin. G207 (1.1 x 10⁹pfu/mL, Neuro Vir, Inc., in PBS) was mixed with human holo-transferrin (Sigma, 5 mg/mL in water) at different ratios in the same manner as that for the Tf-AdLacZ, as described in Example 1. In one set of experiments, HSV was heat-treated by incubating at 37°C for 10 minutes before mixing with Tf. The heat treatment reportedly can inactivate the HSV.

For transduction experiments, 8 x 10⁴ JSQ-3 cells/well were plated in a 24-well plate. 24 hours later the cells were washed once with EMEM without serum, then 0.3 mL EMEM without serum or antibiotics was added to each well. The HSV or Tf-HSV complexes at different ratios of transferrin to virus in 200 µL EMEM were added to the wells, at a MOI=1. After 4 hours incubation at 37°C, 5% CO₂, with mixing by rotating the test tube once every 2 minutes, 0.5 mL EMEM with 20% serum was added to the wells. After 2 days in culture, the cells were washed once with PBS, and lysed in 1X reporter lysis buffer (Promega). The cell lysates were treated with 100 µL of 150 µM 0-nitrophenyl-β-galactopyranoside in 20 mM Tris (pH 7.5) containing 1 mM MgCl₂ and 450 mM β-mercaptoethanol at 37°C for 30 minutes. The reaction was stopped by the addition of 150 µL/well of 1 M Na₂CO₃. The absorbance was measured at 405 nm. Purified β-galactosidase (Boehringer) was used to make a standard curve. The results were expressed as milliUnits (mU) of β-galactosidase equivalent per mg of total protein. The results are shown in Table 1.

**Table 1**

| Transferrin Enhances HSV Transduction Efficiency | | | | | | |
|---|---|---|---|---|---|---|
| Tf/virion | 0 | 500 | 1500 | 5000 | 15000 | 50000 |
| Non-treated* | 318 | 448 | 463 | 483 | 405 | 675 |
| Heat-treated * | 286 | 410 | 436 | 455 | 386 | 640 |

| | | | | | | |
|---|---|---|---|---|---|---|
| * β-galactosidase activity, mU/mg protein | | | | | | |

Transferrin enhances the transduction efficiency of HSV, a replication competent viral vector. When HSV was heat-inactivated, the complexing with Tf still showed enhanced transduction efficiency. At a Tf/virion ratio of 50000 and a MOI=1, Tf-HSV gave greater than two-fold more reporter gene expression than did HSV without Tf targeting. The results demonstrate that the transferrin-targeting strategy can be used for replication competent viral vectors such as HSV.

### Example 9

### Transferrin-Targeted Systemic Adenoviral-Mediated Gene Delivery In Vivo in an MDA-MB-435 Xenograft Nude Mouse Model in Conjunction with the Chemotherapeutic Agent Docetaxel (Taxotere)

To further demonstrate the usefulness of this targeted adenoviral delivery system, a second tumor model, the human breast cancer derived cell line MDA-MB-435, was employed. Additionally, since chemotherapy is often the treatment of choice for breast cancer, this experiment tested the ability of the delivery system of this invention to sensitize established human breast cancer xenograft tumors to the commonly used chemotherapeutic agent docetaxel (Taxotere). Xenografts were induced in 4-6 week old female athymic nude (NCr nu-nu) mice by the subcutaneous injection of 2.5 x 10⁶ MDA-MB-435 cells in the mammary fat pad of each animal. Tumors were allowed to develop to a size of 40-50 mm³. The targeted adenovirus, designated Tf Adp53, was prepared by mixing transferrin with adenovirus carrying DNA encoding wt p53 as described in Example 1. The animals were divided into five groups: (i) Untreated (-) Taxotere; (ii) Taxotere alone, (iii) Untargeted Adp53 (+) Taxotere; (iv) Targeted Adp53 (-) Taxotere; (v) Targeted Adp53 (+) Taxotere. The mice were i.v. injected, via the tail vein, every three to four days with 5 x 10¹⁰ pt/mouse/injection of either targeted (an admixture of Tf ligand and virus was injected) or untargeted (no ligand) Adp53. A total of 12 injections were administered. The day after the initial viral injection, drug treatment was started. The animals were given Taxotere i.v. at a dose of 7.5 mg/kg every three or four days to a total of 11 injections. The tumor sizes were measured weekly in a blinded manner on a total of 6-8 tumors/group. The mean of the tumor volumes per group (mm³) ± Standard Error vs. Time (Days) was plotted (Figure 6). While treatment with the Tf Adp53 alone had no effect, treatment with Taxotere alone or the untargeted Adp53 plus Taxotere induced some growth inhibition indicating a drug effect. However, there was an even more dramatic level of growth inhibition observed in the tumors from the animals receiving Tf-Targeted Ad-p53 in combination with taxotere. These findings show the synergistic effect of the combination treatment and demonstrate not only that the transferrin-targeted Adp53 complex of the invention is effective in multiple human tumor models, but that it can also be used to sensitize tumors to chemotherapeutic agents.

### Example 10

### Transferrin- Targeted Adenoviral-Mediated Gene Expression In Vivo in a DU145 Xenograft Nude Mouse Model

A prostate cancer derived cell line, DU 145, demonstrated improved expression in intratumoral injections. The replication deficient adenovirus serotype 5, that carried the LacZ gene, was used in this example. Athymic nude (nu/nu) mice were subcutaneously injected using Matrigel™ to produce tumors. Tf-Ad-LacZ was prepared as in Example 1. The ratio of Tf/pt was 0.1-0.2 mg/10¹⁰ pt as described in Example 2. The mice had 2 tumors each but only one was injected.

Twenty-four hours after intratumoral injection of 3x10¹⁰ particles/tumor, the tumors were excised, cut into pieces, flash frozen in liquid nitrogen, and were pulverized in a Bessman tissue pulverizer. β-Galactosidase enzyme activity was measured using the Glacto-Star^{™} chemiluminescent β-Galactosidase assay system from Tropix, Inc. according to the manufacturer's protocol. The results are shown in Figure 7. Tf-Ad-LacZ gave greater than a 3.4-fold increase in β-galactosidase expression as compared to Ad-LacZ. The results demonstrate that the transferrin-targeting strategy can be used for increasing expression in intratumoral injections.

### LIST OF REFERENCES

Aisen P (1998). Met. Ions Biol. Syst. 35:585-631.
Asgari K, et al. (1997). Int. J. Cancer 71:377-382.
Baselga J and Mendelsohn J (1994). Pharmacol. Ther. 64:127-154.
Berkner KL (1988). BioTechniques 6:616-629.
Bischoff JR, et al. (1996). Science 274:373-376.
Bristow RG, et al. (1996). Radiother. Oncol. 40: 197-223.
Cotten M, et al. (1992). Proceedings Natl. Acad Sci. USA 89:6094-6098.
Douglas JT, et al. (1996). Nat. Biotechnol. 14:1574-1578.
Goud B, et al. (1988). Virology 163:251-254.
Hall AR, et al. (1998). Nat. Med. 4:1068-1072.
Heise C, et al. (1997). Nat. Med. 3:639-645.
Isaacs WB, et al. (1991). Cancer Res. 51:4716-4720*.*
Kataoka M, et al. (1998). Cancer Res. 58:4761-4765.
Kirn D, et al. (1998). Nat. Med 4:1341-1342.
Linke SP (1998). Nature 395 :13,15.
Miller N and Vile R (1995). Faseb 9:190-199
Miyamoto T, et al. (1994). Int. J. Oral. Maxillofac. Surg. 23:430-433.
Pirollo KF, et al. (1997). Oncogene 14:1735-1746.
Rogers BE, et al. (1997). Gene Therapy 4:1387-1392.
Roux P, et al. (1989). Proceedings Natl. Acad. Sci. USA 86:9079-9083.
Schwarzenberger P, et al. (1997). J. Virol. 71:8563-8571.
Snitkovsky S and Young JAT (1998) PNAS 95:7063-7068
Wagner E, et al. (1992). Proceedings Natl. Acad. Sci. USA 89:6099-6103.
Walker JR, et al. (1999). Hum. Gene Ther. 10:2237-2243.
Weichselbaum RR, et al. (1988). Int. J. Radiat. Oncol. Biol. Phys. 15:575-579.
Wivel NA and Wilson JM (1998). Hematol. Oncol. Clin. North Am. 12:483-501.
Xu L, et al. (1997). Human Gene Therapy 8:467-475.
Xu L, et al. (1999). Tumor Targeting 4:92-104.
U.S. Patent No. 5,108,921
U.S. Patent No. 5,139,941
U.S. Patent No. 5,288,641
U.S. Patent No. 5,378,457
U.S. Patent No. 5,416,016
U.S. Patent No. 5,521,291
U.S. Patent No. 5,547,932
U.S. Patent No. 5,635,382
U.S. Patent No. 5,693,509
U.S. Patent No. 5,762,938
U.S. Patent No. 5,833,975
WO 92/06180
WO 98/40508

## Claims

1. A cell-targeting ligand-virus complex for delivery of a virus to a target cell within a host animal, wherein said ligand-virus complex consists of a ligand and a virus, and wherein the ligand is non-covalently bound to the virus, and is transferrin, a transferrin receptor antibody, a fragment of a transferrin receptor antibody, EGF or VEGF.

2. The cell-targeting ligand-virus complex according to claim 1 wherein said virus and said ligand are not naturally associated with each other.

3. The cell-targeting ligand-virus complex according to claim 1 or claim 2 wherein said virus is comprised of a therapeutic nucleic acid.

4. The cell-targeting ligand-virus complex according to claim 3 wherein said therapeutic nucleic acid encodes a therapeutic peptide or protein.

5. The cell-targeting ligand-virus complex according to claim 4 wherein said therapeutic nucleic acid encodes wild-type p53.

6. The cell-targeting ligand-virus complex according to any preceding claim wherein said virus is a retrovirus or an adenovirus.

7. The cell-targeting ligand-virus complex according to any one of claims 1 to 5 wherein said virus is selected from the group consisting of adeno-associated virus, herpes simplex virus, cytomegalovirus, vaccinia virus, fowlpoxvirus, canarypoxvirus and Sindbis virus.

8. The cell-targeting ligand-virus complex according to any preceding claim wherein said virus is a chimeric virus, a hybrid virus, or a recombinant virus.

9. The cell-targeting ligand-virus complex according to any preceding claim wherein said cell-targeting ligand is a native protein or a recombinant protein.

10. The cell-targeting ligand-virus complex according to any preceding claim wherein said cell-targeting ligand and said virus are present at a ratio in the range of 100 to 1, 000, 000 ligand molecules per virion.

11. The cell-targeting ligand-virus complex according to claim 10 wherein said cell-targeting ligand and said virus are present at a ratio in the range of 6,700 to 400,000 ligand molecules per virion.

12. The cell-targeting ligand-virus complex according to any preceding claim wherein said cell-targeting ligand and said virus are present at a ratio in the range of 1 µg to 10 mg of said ligand per 10¹⁰ virion.

13. The cell-targeting ligand-virus complex according to claim 12 wherein said cell-targeting ligand and said virus are present at a ratio in the range of 10 µg to 600 µg of said ligand per 10¹⁰ virion.

14. A vector for the systemic delivery of a virus to a target cell, said vector comprising a cell-targeting ligand non-covalently bound to said virus, wherein said vector is preparable by mixing a composition consisting of a cell-targeting ligand with a virus in an aqueous medium and wherein said ligand consists of transferrin, a transferrin receptor antibody, a fragment of a transferrin receptor antibody, EGF or VEGF.

15. The vector according to claim 14 wherein said virus and said ligand are not naturally associated with each other.

16. The vector according to claim 14 or claim 15 wherein said virus is comprised of a therapeutic nucleic acid.

17. The vector according to claim 16 wherein said therapeutic nucleic acid encodes a therapeutic peptide or protein.

18. The vector according to claim 17 wherein said therapeutic nucleic acid encodes wild-type p53.

19. The vector according to any of claims 14 to 18 wherein said virus is a retrovirus or an adenovirus.

20. The vector according to any of claims 14 to 18 wherein said virus is selected from the group consisting of adeno-associated virus, herpes simplex virus, cytomegalovirus, vaccinia virus, fowlpoxvirus, canarypoxvirus and Sindbis virus.

21. The vector according to any of claims 14 to 20 wherein said virus is a chimeric virus, a hybrid virus, or a recombinant virus.

22. The vector according to any of claims 14 to 21 wherein said cell-targeting ligand is a native protein or a recombinant protein.

23. The vector according to any of claims 14 to 22 wherein said cell-targeting ligand and said virus are present at a ratio in the range of 100 to 1,000,000 ligand molecules per virion.

24. The vector according to claim 23 wherein said cell-targeting ligand and said virus are present at a ratio in the range of 6,700 to 400,000 ligand molecules per virion.

25. The vector according to any of claims 14 to 24 wherein said cell-targeting ligand and said virus are present at a ratio in the range of 1 µg to 10 mg of said ligand per 10¹⁰ virion.

26. The vector according to claim, 25 wherein said cell-targeting ligand and said virus are present at a ratio in the range of 10 µg to 600 µg of said ligand per 10¹⁰ virion.

27. A method for preparing a vector for the systemic delivery of a virus to a target cell, said vector comprising a cell-targeting ligand non-covalently bound to said virus, comprising mixing a composition consisting of said cell-targeting ligand with said virus in an aqueous medium, whereby said ligand non-covalently binds to said virus, wherein said ligand consists of transferrin, a transferrin receptor antibody, a fragment of a transferrin receptor antibody, EGF or VEGF.

28. The method of claim 27 wherein said aqueous solution includes one or more of a buffering agent, an osmolarity adjusting agent, or an antibiotic.

29. The cell-targeting ligand-virus complex according to any one of claims 1 to 13 for use as a medicament.

30. The vector according to any one of claims 14 to 28 for use as a medicament.

31. Use of a virus for the manufacture of a therapeutic agent comprising a vector which comprises a cell targeting ligand-virus complex, for delivery of said virus to a target cell within an animal, wherein said ligand-virus complex consists of a ligand and a virus, and wherein the ligand is non-covalently bound to the virus and is transferrin, a transferrin receptor antibody, a fragment of a transferrin receptor antibody, EGF or VEGF.

32. Use according to claim 31 wherein said animal is a human.

33. Use according to claim 31 or claim 32 wherein said therapeutic agent is administered systemically.

34. Use according to claim 31 or claim 32 wherein said therapeutic agent is administered parenterally.

35. Use according to claim 31 or claim 32 wherein said therapeutic agent is administered intravenously or intra-arterially.

36. Use according to claim 31 or claim 32 wherein said therapeutic agent is administered intratumorally.

37. Use according to any of claims 31 to 36 wherein said vector encodes wild-type p53.

38. Use according to any of claims 31 to 37 wherein said therapeutic agent is administered to an animal receiving chemotherapy in addition to said therapeutic agent.

39. Use according to any of claims 31 to 37 wherein said therapeutic agent is administered to an animal receiving radiation treatment in addition to said therapeutic agent.

40. Use according to any of claims 31 to 39 wherein said animal has cancer of the head and neck, bladder, breast, thyroid, ovary, brain, prostate, a melanoma or a lymphoma.

41. Use according to any of claims 31 to 33 and claims 37 to 40 wherein said virus is comprised of a nucleic acid encoding wild-type p53, further wherein said cell-targeting ligand is transferrin, and further whereby said therapeutic agent is administered systemically.

42. Use according to claim 33 or claim 36, wherein the target cell is a cancer cell selected from a head and neck cancer, bladder cancer, breast cancer, thyroid cancer, ovarian cancer, prostate cancer, melanoma and lymphoma;
and the vector consists essentially of the virus and binds directly to a receptor which is overexpressed in the cancer cell.

43. Use according to claim 42, wherein the virus sensitizes the cancer cell to radiation or chemotherapy.

44. Use according to claim 43, wherein the virus comprises a nucleic acid of interest, expression of which in the cancer cell sensitizes the cancer cell to radiation or chemotherapy.

45. Use according to claim 40, wherein the virus comprises a nucleic acid which, when expressed in cancer cells, sensitizes the cell to radiation or chemotherapy;
the ligand binds directly to a receptor on the cancer cell; and
the vector consists essentially of the virus and the ligand.

46. A vector for use in delivery of a virus to a target cell within an animal, wherein the vector comprises a cell targeting ligand-virus complex, wherein said ligand-virus complex consists of a ligand and a virus, and wherein the ligand is non-covalently bound to the virus and is transferrin, a transferrin receptor antibody, a fragment of a transferrin receptor antibody, EGF or VEGF.

## Patentansprüche

1. Zell-Targeting-Ligand/Virus-Komplex zum Transport eines Virus zu einer Zielzelle in einem Wirtstier, wobei der Ligand/Virus-Komplex aus einem Liganden und einem Virus besteht, und wobei der Ligand nichtkovalent an das Virus gebunden ist und Transferrin, ein Transferrin-Rezeptor-Antikörper, ein Fragment eines Transferrin-Rezeptor-Antikörpers, EGF oder VEGF ist.

2. Zell-Targeting-Ligand/Virus-Komplex nach Anspruch 1, wobei das Virus und der Ligand von Natur aus nicht miteinander assoziiert sind.

3. Zell-Targeting-Ligand/Virus-Komplex nach Anspruch 1 oder Anspruch 2, wobei das Virus eine therapeutische Nucleinsäure umfasst.

4. Zell-Targeting-Ligand/Virus-Komplex nach Anspruch 3, wobei die therapeutische Nucleinsäure für ein therapeutisches Peptid oder Protein kodiert.

5. Zell-Targeting-Ligand/Virus-Komplex nach Anspruch 4, wobei die therapeutische Nucleinsäure für Wildtyp-p53 kodiert.

6. Zell-Targeting-Ligand/Virus-Komplex nach einem der vorstehenden Ansprüche, wobei das Virus ein Retrovirus oder ein Adenovirus ist.

7. Zell-Targeting-Ligand/Virus-Komplex nach einem der Ansprüche 1 bis 5, wobei das Virus ausgewählt ist aus der Gruppe bestehend aus adenoassoziiertem Virus, Herpes-simplex-Virus, Zytomegalievirus, Vacciniavirus, Geflügelpockenvirus, Kanarienpockenvirus und Sindbis-Virus.

8. Zell-Targeting-Ligand/Virus-Komplex nach einem der vorstehenden Ansprüche, wobei das Virus ein chimäres Virus, ein Hybridvirus oder ein rekombinantes Virus ist.

9. Zell-Targeting-Ligand/Virus-Komplex nach einem der vorstehenden Ansprüche, wobei der Zell-Targeting-Ligand ein natives Protein oder ein rekombinantes Protein ist.

10. Zell-Targeting-Ligand/Virus-Komplex nach einem der vorstehenden Ansprüche, wobei der Zell-Targeting-Ligand und das Virus in einem Verhältnis im Bereich von 100 bis 1.000.000 Ligandenmolekülen pro Virion vorhanden sind.

11. Zell-Targeting-Ligand/Virus-Komplex nach Anspruch 10, wobei der Zell-Targeting-Ligand und das Virus in einem Verhältnis von 6.700 bis 400.000 Ligandenmoleküle pro Virion vorhanden sind.

12. Zell-Targeting-Ligand/Virus-Komplex nach einem der vorstehenden Ansprüche, wobei der Zell-Targeting-Ligand und das Virus in einem Verhältnis im Bereich von 1 µg bis 10 mg des Liganden pro 10¹⁰ Virionen vorhanden sind.

13. Zell-Targeting-Ligand/Virus-Komplex nach Anspruch 12, wobei der Zell-Targeting-Ligand und das Virus in einem Verhältnis im Bereich von 10 µg bis 600 µg des Liganden pro 10¹⁰ Virionen vorhanden sind.

14. Vektor zum systemischen Transport eines Virus zu einer Zielzelle, wobei der Vektor einen nichtkovalent an das Virus gebundenen Zell-Targeting-Liganden umfasst, wobei der Vektor durch Mischen einer Zusammensetzung aus einem Zell-Targeting-Liganden mit einem Virus in einem wässrigen Medium herstellbar ist und wobei der Ligand aus Transferrin, einem Transferrin-Rezeptor-Antikörper, einem Fragment eines Transferrin-Rezeptor-Antikörpers, EGF oder VEGF besteht.

15. Vektor nach Anspruch 14, wobei das Virus und der Ligand von Natur aus nicht miteinander assoziiert sind.

16. Vektor nach Anspruch 14 oder Anspruch 15, wobei das Virus eine therapeutische Nucleinsäure umfasst.

17. Vektor nach Anspruch 16, wobei die therapeutische Nucleinsäure für ein therapeutisches Peptid oder Protein kodiert.

18. Vektor nach Anspruch 17, wobei die therapeutische Nucleinsäure für Wildtyp-p53 kodiert.

19. Vektor nach einem der Ansprüche 14 bis 18, wobei das Virus ein Retrovirus oder ein Adenovirus ist.

20. Vektor nach einem der Ansprüche 14 bis 18, wobei das Virus aus der Gruppe ausgewählt ist, bestehend aus adenoassoziiertem Virus, Herpes-simplex-Virus, Zytomegalievirus, Vacciniavirus, Geflügelpockenvirus, Kanarienpockenvirus und Sindbis-Virus.

21. Vektor nach einem der Ansprüche 14 bis 20, wobei das Virus ein chimäres Virus, ein Hybridvirus oder ein rekombinantes Virus ist.

22. Vektor nach einem der Ansprüche 14 bis 21, wobei der Zell-Targeting-Ligand ein natives Protein oder ein rekombinantes Protein ist.

23. Vektor nach einem der Ansprüche 14 bis 22, wobei der Zell-Targeting-Ligand und das Virus in einem Verhältnis im Bereich von 100 bis 1.000.000 Ligandenmoleküle pro Virion vorhanden sind.

24. Vektor nach Anspruch 23, wobei der Zell-Targeting-Ligand und das Virus in einem Verhältnis im Bereich von 6.700 bis 400.000 Ligandenmoleküle pro Virion vorhanden sind.

25. Vektor nach einem der Ansprüche 14 bis 24, wobei der Zell-Targeting-Ligand und das Virus in einem Verhältnis im Bereich von 1 µg bis 10 mg des Liganden pro 10¹⁰ Virionen vorhanden sind.

26. Vektor nach Anspruch 25, wobei der Zell-Targeting-Ligand und das Virus in einem Verhältnis im Bereich von 10 µg bis 600 µg des Liganden pro 10¹⁰ Virionen vorhanden sind.

27. Verfahren zur Herstellung eines Vektors zum systemischen Transport eines Virus zu einer Zielzelle, wobei der Vektor einen nichtkovalent an das Virus gebundenen Zell-Targeting-Liganden umfasst, umfassend das Mischen einer Zusammensetzung aus dem Zell-Targeting-Liganden mit dem Virus in einem wässrigen Medium, wobei der Ligand aus Transferrin, einem Transferrin-Rezeptor-Antikörper, einem Fragment eines Transferrin-Rezeptor-Antikörpers, EGF oder VEGF besteht.

28. Verfahren gemäß Anspruch 27, wobei die wässrige Lösung eines oder mehrere aus einem Puffer, einem Mittel zur Einstellung der Osmolarität und einem Antibiotikum umfasst.

29. Zell-Targeting-Ligand/Virus-Kotnplex nach einem der Ansprüche 1 bis 13 zur Verwendung als Medikament.

30. Vektor nach einem der Ansprüche 14 bis 28 zur Verwendung als Arzneimittel.

31. Verwendung eines Virus zur Herstellung eines therapeutischen Mittels, das einen Vektor umfasst, der einen Zell-Targeting-Ligand/Virus-Komplex umfasst, zum Transport des Virus zu einer Zielzelle in einem Tier, wobei der Ligand/Virus-Komplex aus einem Liganden und einem Virus besteht, und wobei der Ligand nichtkovalent an das Virus gebunden ist und Transferrin, ein Transferrin-Rezeptor-Antikörper, ein Fragment eines Transferrin-Rezeptor-Antikörpers, EGF oder VEGF ist.

32. Verwendung nach Anspruch 31, wobei das Tier ein Mensch ist.

33. Verwendung nach Anspruch 31 oder 32, wobei das therapeutische Mittel systemisch verabreicht wird.

34. Verwendung nach Anspruch 31 oder 32, wobei das therapeutische Mittel parenteral verabreicht wird.

35. Verwendung nach Anspruch 31 oder 32, wobei das therapeutische Mittel intravenös oder intraarteriell verabreicht wird.

36. Verwendung nach Anspruch 31 oder Anspruch 32, wobei das therapeutische Mittel intratumoral verabreicht wird.

37. Verwendung nach einem der Ansprüche 31 bis 36, wobei der Vektor für Wildtyp-p53 kodiert.

38. Verwendung nach einem der Ansprüche 31 bis 37, wobei das Therapeutikum einem Tier verabreicht wird, das zusätzlich zu diesem Therapeutikum eine Chemotherapie erhält.

39. Verwendung nach einem der Ansprüche 31 bis 37, wobei das Therapeutikum einem Tier verabreicht wird, das zusätzlich zu diesem Therapeutikum eine Strahlentherapie erhält.

40. Verwendung nach einem der Ansprüche 31 bis 39, wobei das Tier Kopf- und Halskrebs, Blasenkrebs, Brustkrebs, Schilddrüsenkrebs, Eierstockkrebs, Hirnkrebs, Prostatakrebs, ein Melanom oder ein Lymphom hat.

41. Verwendung nach einem der Ansprüche 31 bis 33 und Ansprüche 37 bis 40, wobei das Virus eine Nucleinsäure umfasst, die für Wildtyp-p53 kodiert, wobei weiters der Zell-Targeting-Ligand Transferrin ist, und wobei weiters das Therapeutikum systemisch verabreicht wird.

42. Verwendung nach Anspruch 33 oder Anspruch 36, wobei die Zielzelle eine Krebszelle ist, die aus Kopf- und Halskrebs, Blasenkrebs, Brustkrebs, Schilddrüsenkrebs, Eierstockkrebs, Prostatakrebs, einem Melanom und einem Lymphom ausgewählt ist; und der Vektor im Wesentlichen aus dem Virus besteht und direkt an einen Rezeptor bindet, der in der Krebszelle überexprimiert wird.

43. Verwendung nach Anspruch 42, wobei das Virus die Krebszelle für Strahlung oder Chemotherapie sensibilisiert.

44. Verwendung nach Anspruch 43, wobei das Virus eine Nucleinsäure von Interesse umfasst, deren Expression in der Krebszelle die Krebszelle für Strahlung oder Chemotherapie sensibilisiert.

45. Verwendung nach Anspruch 40, wobei das Virus eine Nucleinsäure umfasst, die bei ihrer Expression in Krebszellen die Zelle für Strahlung oder Chemotherapie sensibilisiert;
der Ligand direkt an einen Rezeptor auf der Krebszelle bindet; und
der Vektor im Wesentlichen aus dem Virus und dem Liganden besteht.

46. Vektor zur Verwendung beim Transport eines Virus zu einer Zielzelle in einem Tier, wobei der Vektor einen Zell-Targeting-Ligand/Virus-Komplex umfasst, wobei der Ligand/Virus-Komplex aus einem Liganden und einem Virus besteht, und wobei der Ligand nichtkovalent an das Virus gebunden ist und Transferrin, ein Transferrin-Rezeptor-Antikörper, ein Fragment eines Transferrin-Rezeptor-Antikörpers, EGF oder VEGF ist.

## Revendications

1. Complexe virus/ligand ciblant les cellules destiné à administrer un virus à une cellule cible à l'intérieur d'un animal hôte, ledit complexe virus/ligand consistant en un ligand et un virus, le ligand étant lié de manière non covalente au virus, et le ligand étant la transferrine, un anticorps du récepteur de la transferrine, un fragment d'anticorps du récepteur de la transferrine, l'EGF ou le VEGF.

2. Complexe virus/ligand ciblant les cellules selon la revendication 1, dans lequel ledit virus et ledit ligand ne sont pas naturellement associés l'un à l'autre.

3. Complexe virus/ligand ciblant les cellules selon la revendication 1 ou la revendication 2, dans lequel ledit virus comprend un acide nucléique thérapeutique.

4. Complexe virus/ligand ciblant les cellules selon la revendication 3, dans lequel ledit acide nucléique thérapeutique code pour un peptide ou une protéine thérapeutique.

5. Complexe virus/ligand ciblant les cellules selon la revendication 4, dans lequel ledit acide nucléique thérapeutique code pour un p53 de type sauvage.

6. Complexe virus/ligand ciblant les cellules selon l'une quelconque des revendications précédentes, dans lequel ledit virus est un rétrovirus ou un adénovirus.

7. Complexe virus/ligand ciblant les cellules selon l'une quelconque des revendications 1 à 5, dans lequel ledit virus est choisi dans le groupe comprenant un virus adéno-associé, un virus de l'herpès simplex, un cytomégalovirus, un virus de la vaccine, un virus de la variole aviaire, un virus de la variole du canari et un virus Sindbis.

8. Complexe virus/ligand ciblant les cellules selon l'une quelconque des revendications précédentes, dans lequel ledit virus est un virus chimère, un virus hybride, ou un virus recombinant.

9. Complexe virus/ligand ciblant les cellules selon l'une quelconque des revendications précédentes, dans lequel ledit ligand ciblant les cellules est une protéine native ou une protéine recombinante.

10. Complexe virus/ligand ciblant les cellules selon l'une quelconque des revendications précédentes, dans lequel ledit ligand ciblant les cellules et ledit virus sont présents à un rapport dans la plage allant de 100 à 1 000 000 molécules de ligand par virion.

11. Complexe virus/ligand ciblant les cellules selon la revendication 10, dans lequel ledit ligand ciblant les cellules et ledit virus sont présents à un rapport dans la plage allant de 6700 à 400 000 molécules de ligand par virion.

12. Complexe virus/ligand ciblant les cellules selon l'une quelconque des revendications précédentes, dans lequel ledit ligand ciblant les cellules et ledit virus sont présents à un rapport dans la plage allant de 1 µg à 10 mg dudit ligand pour 10¹⁰ virions.

13. Complexe virus/ligand ciblant les cellules selon la revendication 12, dans lequel ledit ligand ciblant les cellules et ledit virus sont présents à un rapport dans la plage allant de 10 µg à 600 µg dudit ligand pour 10¹⁰ virions.

14. Vecteur destiné à l'administration systémique d'un virus à une cellule cible, ledit vecteur comprenant un ligand ciblant les cellules qui est lié de manière non covalente au dit virus, ledit vecteur pouvant être préparé en mélangeant une composition consistant en un ligand ciblant les cellules avec un virus dans un milieu aqueux et ledit ligand consistant en la transferrine, un anticorps du récepteur de la transferrine, un fragment d'anticorps du récepteur de la transferrine, l'EGF ou le VEGF.

15. Vecteur selon la revendication 14, dans lequel ledit virus et ledit ligand ne sont pas naturellement associés l'un à l'autre.

16. Vecteur selon la revendication 14 ou la revendication 15, dans lequel ledit virus comprend un acide nucléique thérapeutique.

17. Vecteur selon la revendication 16, dans lequel ledit acide nucléique thérapeutique code pour un peptide ou une protéine thérapeutique.

18. Vecteur selon la revendication 17, dans lequel ledit acide nucléique thérapeutique code pour un p53 de type sauvage.

19. Vecteur selon l'une quelconque des revendications 14 à 18, dans lequel ledit virus est un rétrovirus ou un adénovirus.

20. Vecteur selon l'une quelconque des revendications 14 à 18, dans lequel ledit virus est choisi dans le groupe comprenant un virus adéno-associé, un virus de l'herpès simplex, un cytomégalovirus, un virus de la vaccine, un virus de la variole aviaire, un virus de la variole du canari et un virus Sindbis.

21. Vecteur selon l'une quelconque des revendications 14 à 20, dans lequel ledit virus est un virus chimère, un virus hybride, ou un virus recombinant.

22. Vecteur selon l'une quelconque des revendications 14 à 21, dans lequel ledit ligand ciblant les cellules est une protéine native ou une protéine recombinante.

23. Vecteur selon l'une quelconque des revendications 14 à 22, dans lequel ledit ligand ciblant les cellules et ledit virus sont présents à un rapport dans la plage allant de 100 à 1 000 000 molécules de ligand par virion.

24. Vecteur selon la revendication 23, dans lequel ledit ligand ciblant les cellules et ledit virus sont présents à un rapport dans la plage allant de 6700 à 400 000 molécules de ligand par virion.

25. Vecteur selon l'une quelconque des revendications 14 à 24, dans lequel ledit ligand ciblant les cellules et ledit virus sont présents à un rapport dans la plage allant de 1 µg à 10 mg dudit ligand pour 10¹⁰ virions.

26. Vecteur selon la revendication 25, dans lequel ledit ligand ciblant les cellules et ledit virus sont présents à un rapport dans la plage allant de 10 µg à 600 µg dudit ligand pour 10¹⁰ virions.

27. Procédé de préparation d'un vecteur destiné à l'administration systémique d'un virus à une cellule cible, ledit vecteur comprenant un ligand ciblant les cellules lié de manière non covalente au dit virus, ledit procédé comprenant l'étape consistant à mélanger une composition consistant en ledit ligand ciblant les cellules avec ledit virus dans un milieu aqueux, ledit ligand se liant de manière non covalente au dit virus, ledit ligand consistant en de la transferrine, un anticorps du récepteur de la transferrine, un fragment d'anticorps du récepteur de la transferrine, l'EGF ou le VEGF.

28. Procédé selon la revendication 27, dans lequel ladite solution aqueuse comprend un ou plusieurs parmi un agent tampon, un agent ajustant l'osmolarité, ou un antibiotique.

29. Complexe virus/ligand ciblant les cellules selon l'une quelconque des revendications 1 à 13, destiné à être utilisé comme médicament.

30. Vecteur selon l'une quelconque des revendications 14 à 28, destiné à être utilisé comme médicament.

31. Utilisation d'un virus dans la fabrication d'un agent thérapeutique comprenant un vecteur qui comprend un complexe virus/ligand ciblant les cellules, pour administrer ledit virus à une cellule cible à l'intérieur d'un animal, dans laquelle ledit complexe virus/ligand consiste en un ligand et un virus, et ledit ligand est lié de manière non covalente au virus et ledit ligand est la transferrine, un anticorps du récepteur de la transferrine, un fragment d'anticorps du récepteur de la transferrine, l'EGF ou le VEGF.

32. Utilisation selon la revendication 31, dans laquelle ledit animal est un humain.

33. Utilisation selon la revendication 31 ou la revendication 32, dans laquelle ledit agent thérapeutique est administré de manière systémique.

34. Utilisation selon la revendication 31 ou la revendication 32, dans laquelle ledit agent thérapeutique est administré de manière parentérale.

35. Utilisation selon la revendication 31 ou la revendication 32, dans laquelle ledit agent thérapeutique est administré par voie intraveineuse ou intra-artérielle.

36. Utilisation selon la revendication 31 ou la revendication 32, dans laquelle ledit agent thérapeutique est administré par voie intra-tumorale.

37. Utilisation selon l'une quelconque des revendications 31 à 36, dans laquelle ledit vecteur code pour un p53 de type sauvage.

38. Utilisation selon l'une quelconque des revendications 31 à 37, dans laquelle ledit agent thérapeutique est administré à un animal recevant une chimiothérapie en plus dudit agent thérapeutique.

39. Utilisation selon l'une quelconque des revendications 31 à 37, dans laquelle ledit agent thérapeutique est administré à un animal recevant un traitement par rayonnements en plus dudit agent thérapeutique.

40. Utilisation selon l'une quelconque des revendications 31 à 39, dans laquelle ledit animal a un cancer de la tête et du cou, de la vessie, du sein, de la thyroïde, de l'ovaire, du cerveau, de la prostate, un mélanome ou un lymphome.

41. Utilisation selon l'une quelconque des revendications 31 à 33 et des revendications 37 à 40, dans laquelle ledit virus comprend un acide nucléique codant pour un p53 de type sauvage, dans laquelle, en outre, ledit ligand ciblant les cellules est la transferrine, et dans laquelle, en outre, ledit agent thérapeutique est administré de manière systémique.

42. Utilisation selon la revendication 33 ou la revendication 36, dans laquelle la cellule cible est une cellule cancéreuse choisie parmi une cellule du cancer de la tête et du cou, du cancer de la vessie, du cancer du sein, du cancer de la thyroïde, du cancer de l'ovaire, du cancer de la prostate, d'un mélanome et d'un lymphome ;
et le vecteur consiste essentiellement en le virus et se lie directement à un récepteur qui est surexprimé dans la cellule cancéreuse.

43. Utilisation selon la revendication 42, dans laquelle le virus sensibilise la cellule cancéreuse aux rayonnements ou à la chimiothérapie.

44. Utilisation selon la revendication 43, dans laquelle le virus comprend un acide nucléique intéressant, dont l'expression dans la cellule cancéreuse sensibilise la cellule cancéreuse aux rayonnements ou à la chimiothérapie.

45. Utilisation selon la revendication 40, dans laquelle le virus comprend un acide nucléique qui, lorsqu'il est exprimé dans les cellules cancéreuses, sensibilise la cellule aux rayonnements ou à la chimiothérapie,
le ligand se liant directement à un récepteur sur la cellule cancéreuse ; et
le vecteur consistant essentiellement en le virus et le ligand.

46. Vecteur destiné à être utilisé pour l'administration d'un virus à une cellule cible à l'intérieur d'un animal, le vecteur comprenant un complexe virus/ligand ciblant les cellules, ledit complexe virus/ligand consistant en un ligand et un virus, et le ligand étant lié de manière non covalente au virus et le ligand étant la transferrine, un anticorps du récepteur de la transferrine, un fragment d'anticorps du récepteur de la transferrine, l'EGF ou le VEGF.
